# EUROPEAN PATENT APPLICATION

(11) **EP 4 242 654 A1**
(43) Date of publication of application: **13.09.2023**
(21) Application number: 22161293.0
(22) Date of filing: 10.03.2022
(51) Int. Cl.: G01N 33/53, B01L 3/00, G01N 33/543

(54) **A METHOD FOR RAPID IMMUNOASSAYS USING A THREE-DIMENSIONAL MICROFLUIDIC CAPILLARY SYSTEM**

(71) Applicant: PAUL SCHERRER INSTITUT, 5232 Villigen PSI (CH)
(72) Inventor: EKINCI, Yasin, 8046 Zürich (CH); MORTELMANS, Thomas, 5200 Brugg (CH)
(74) Representative: Fischer, Michael

(57) **Abstract**

The present invention relates to a method for rapid immunoassays using a three-dimensional microfluidic capillary system, wherein the 3D microfluidic capillary system has a 3D fluidic trapping section having its cross-section area diminishing in the flow direction of the fluid through the system and wherein support beads or biofunctionalized particles used in said method have a dimension to enable them to be size-dependently immobilized at a predefined position in the fluidic trapping section.

## Description

The present invention relates to a method for rapid immunoassays using a three-dimensional microfluidic capillary system.

Plasma contains a large variety of proteins that are essential to maintain bodily hemostasis and can be used as a valuable source of biomarkers. Its composition can reveal infections, autoimmune diseases and helps to identify many other disorders. To this extent, it is a commonly used biofluid in point-of-care (POC) tests, such as lateral flow assays (LFAs), to provide insights into a person's immunity against different diseases. It also accounts for 55% of whole blood volume. The remaining volume consists of cells, the most abundant of which are the red blood cells (RBCs) or erythrocytes. These are biconcave disks with a thickness of 2.5 µm at the edges and 1 µm at the center.

However, as the readout mechanism of POC-tests is mostly colorimetric, the intense red color of RBCs interferes with the measurement. Numerous methods, therefore, exist to remove all cellular material from whole blood prior to performing immunoassays with a colorimetric readout. These include centrifugation, agglutination, or whole blood filtration. In POC devices, the last two are often combined with membranebased dead-end filtration techniques. Nevertheless, a major downside of using such membranes is that they do not only retain RBCs, but due to electrostatic effects, also retain up to 30% of potential plasma biomarkers. Furthermore, such filtration could possibly cause hemolysis, ostensibly increasing analyte concentration. The integration of filter membranes in micro- and nanofluidic systems is also not straightforward and poses significant fabrication challenges. To overcome the aforementioned drawbacks, it is possible to integrate on-chip blood plasma separation by exploiting various fluidic principles, such as deterministic lateral displacement or the Zweifach-Fung effect. Yet, this necessitates meticulous control over the flow rate by means of active pumping, significantly hampering the applicability of such assays in a POC setting.

It is, therefore, the objective of the present invention to provide a method for rapid immunoassays using a three-dimensional microfluidic capillary system without the need for sample pre-processing or active pumping strategies.

This objective is achieved according to the present invention by a method of rapid on-bead immunoassays using a three-dimensional microfluidic capillary system, said method comprising the steps of:
a) providing the 3D microfluidic capillary system having a 3D fluidic trapping section having its cross-section area diminishing in the flow direction of the fluid through the system,
b) providing an ensemble of support beads being covered with a functionalized layer or biofunctionalized particles, wherein the support beads or the biofunctionalized particles having a dimension to enable them to be size-dependently immobilized at a predefined position in the fluidic trapping section and wherein the functionalized layer or the biofunctionalized particles are chosen to bind one or more specimens of biochemical compounds;
c) conveying the support beads or the biofunctionalized particles into the fluidic trapping section, thereby immobilizing the support beads or the biofunctionalized particles at the predefined position;
d) conveying a probe fluid comprising various specimens of biochemical compounds through the fluidic trapping section, wherein one or more of the various specimens bind to the functionalized layer or to the biofunctionalized particles;
e) conveying optionally a buffer fluid to flush the channels to remove the excessive unbound molecules
   and
f) detecting qualitatively or quantitatively the amount of the one or more specimens that are bound to the functionalized layer being immobilized with the support beads bearing said functionalized layer or to the immobilized biofunctionalized particles.

Thus, the present method allows by use of the tapered fluidic trapping section that (antigen) bio-functionalized particles or the covered support beads are size-dependently immobilized before using it for the application of sample fluids, such as the antibody-containing sample. The pre-trapped beads or particles serve as the substrate for on-bead or on-particle linked immunosorbent assays, such as fluorescence-linked immunosorbent assays. Moreover, by leveraging the shallow device dimensions of the device, it is shown that device readout is possible in less than 10 min and does not require washing steps due to the small detection volume inside the microfluidic channels.

In a preferred embodiment of the present invention, the fluidic trapping section can be dried after the trapping of the support beads or of the biofunctionalized particles. This measure allows to prepare the 3D fluidic capillary system well in advance to the effective immunoassays. Thus, the system can be configured with the immobilized beads or particles and then shipped to the respective laboratories to execute the immunoassay investigations.

Typically, the specimen of a biochemical compound is a peptide, lipide and/or a protein and/or antibody.

In a further preferred embodiment of the present invention, the probe fluid can be whole blood. Specific examples of immunoassays with this kind of probe fluid are described below in the specific description of the attached drawing in more detail.

In order to execute a multiplexed immunoassay investigation, a further preferred embodiment of the present invention can provide for distinct biofunctionalized particles or layered support beads of different sizes, thus enabling an on-chip multiplexed detection, such as antibody detection, against different antigen targets or diseases in the sample fluid, preferably in whole blood samples.

Preferred embodiments of the present invention are hereinafter described in more detail with reference to the attached drawing which depicts in:
- Figure 1: schematically the influence of the channel topography in a 3D fluidic capillary device on whole blood capillary flow; and
- Figure 2: schematically the principles of whole blood rapid and multiplexed immunoassay methods.

Here, the present invention proposes as a viable example the use of poly(methyl methacrylate) (PMMA) three-dimensional (3D) microfluidic device which is capable of performing fluorescence-linked immunosorbent on-bead and on-chip immunoassays, particularly directly in whole blood, without the need for sample pre-processing or active pumping strategies. The device has a 3D channel profile which enables size-dependent immobilization of biofunctionalized particles. The particles are pre-immobilized at a given position in the 3D channel and whole blood is subsequently flushed through the channel in a second loading step. In the first section, the optimization of the channel topography is outlined on the one hand to efficiently pre-trap the particles and, on the other hand, to ensure the easy flow of other components of noninterest comprised in the probe fluid through the device, such as whole blood and its erythrocytes. A suitable channel profile disclosed hereinafter can be used that enables the capillary-driven probe fluid flow, such as the whole blood flow, to perform on-chip immunoassays. As a readout technique, an indirect fluorescence immunoassay can be used and, in doing so, the optical influence of RBCs is reduced. Furthermore, the incubation of the different immunoassays reagents happens concurrently on-chip and because of the reduced channel height, their diffusion distance is significantly reduced in comparison to incubation in standard 96-well plates.

Consequently, the time it takes for an assay to be read out is significantly shortened while simultaneously avoiding cumbersome sample handling steps, such as washing and sequential addition of assay reagents. It is shown that by pre-trapping two or even more sizes of bio-functionalized beads, it is possible to perform multiplexed detection of antibodies against different antigen targets. Due to the size-dependent particle separation, deposition of such bead-testing lines is straightforward in comparison to LFAs. The device is purely driven by capillary forces and, therefore, self-powered. This makes its operation uncomplicated and paves the way for extensive applications in POC settings.

The microfluidic devices were designed using grayscale e-beam lithography fabrication procedure, and its details can be found in our earlier work. After the fabrication of the master structure with the e-beam, a negative copy needs to enable nanoimprint lithography of the microfluidic structures. To do so, a borosilicate wafer was cleaned in acetone and isopropyl alcohol (IPA). Afterwards, the wafer was blow-dried with nitrogen and its surface was activated with oxygen plasma. The activated wafer was spin-coated with an adhesion promoter (Ormoprime, Microresist) and was baked on a hotplate. Subsequently, 1 mL of a photo-curable polymer (GMN PS-90, Optool) was pipetted onto the master structure. The coated borosilicate wafer was then carefully placed on top of the e-beam structure. The GMN-PS90 was left to spread between the glass wafer and the master wafer. Hereafter, the polymer was exposed to UV light to harden polymer (daughter stamp) could easily be removed from the e-beam master.

The daughter stamp was used to hot emboss the negative structures into a 1-mm-thick PMMA sheet. To do so, the daughter stamp and the PMMA sheet were put into a hot embossing chamber. A silicon wafer with an anti-adhesion coating was placed onto the backside of the PMMA sheet, followed by a poly(amide)- poly(dimethyl siloxane) - poly(amide) sandwich to equalize the pressure on the surface of the PMMA film. Afterwards, a touch force of 300 N was applied and the imprinting chamber was heated to 160 °C at 9 °C/min. Afterwards, the force increased to 10 000 N, where it was kept for 15 min. The chamber was then cooled to a demolding temperature of 60 °C before the PMMA sheet was removed from the imprinting chamber. The topography of the devices was investigated via optical and contact profilometry.

After hot-embossing, the PMMA sheets were activated with oxygen plasma which renders the PMMA sheet hydrophilic, enabling the spin-coating of a water-soluble protection layer (10% Dextran in MilliQ; 66kDa Roth Industries). The PMMA sheet was cut into two chips, each containing 9 devices. Afterwards, the protective layer was dissolved by submerging the single chips in deionized water for 15 min. The chips were blow dried with nitrogen and placed under vacuum for a minimum period of 10 min.

Afterwards, the patterned PMMA was activated together with an unpatterned 200-µm-thick optical-grade film by UV/ozone irradiation. The surfaces of both PMMA films were spin-coated with poly(vinyl alcohol) (PVA; 0.5% in phosphate buffered saline (PBS) with a pH of 7.4) to prevent unspecific interactions between biomolecules and the surface of the PMMA²⁹. Excess PVA was removed by spin-washing with deionized water. Once coated, both PMMA surfaces were thermally bonded at 750 N and 45 °C for 1 min.

The filling of the capillary pump was investigated using a light and fluorescence microscope. A 5x objective with a numerical aperture of 0.12 was used to have a large field of view. The influence of the wedge profile and the blood concentration on the flow properties of the 3D microfluidic device was investigated by using nine unique channel profiles, in combination with three concentrations of ethylenediaminetetraacetic acid (EDTA) - treated rabbit whole blood (Undiluted, 1:2, 2:40; Envion). To simplify the tracking of the fluid, a fluorescent dye (ATTO488) was added 1:10 to all three solutions to reach a final concentration of 125 µM. During device loading, a time-lapse series was acquired to monitor the evolution of the flow velocity during filling. Also, five minutes after loading the device, a stitched image was taken to assess the filling factor of the capillary pump. The images were analyzed with a custom Python script, where image segmentation was performed to identify the number of fluorescent pixels and determine the corresponding volume. These data were subsequently used to obtain the average flow rate during capillary filling.

Streptavidin-coated 2.8 µm magnetic beads (Dynabeads^{™} M-280 Streptavidin, ThermoFisher Scientific, 11205D) were washed three times in PBS. Afterwards, the desired number of beads was resuspended in a PBS solution containing 160 pmol of biotinylated BSA per µg beads (Pierce^{™} Bovine Serum Albumin, Biotinylated, ThermoFisher Scientific, 29130). The proteinbead mixture was incubated at 600 rpm and 25 °C for 2 h with periodic vortexing every 30 min and finally washed three times with PBS. The functionalized beads were diluted in deionized water and applied to the inlet of the microfluidic device so that the trapped beads formed a discernable line in the trapping region upon complete filling of the capillary pump. The filled devices were dried in a vacuum chamber for 1.5 h.

To investigate the time evolution of the signal when loading the device, a 2µl PBS-droplet containing 130 nM diluted anti-BSA antibody (Bethyl Laboratories; Bovine Albumin Polyclonal Antibody, A10-127A) and 50 nM Cy5 donkey anti-rabbit antibody was applied onto the inlet of the device. The fluorescence of the beads was monitored over time at 45-second intervals to minimize bleaching, for a total time of 390 s. As a negative control, a PBS solution solely containing Cy5 donkey anti-rabbit antibody was used and monitored over the same amount of time. The fluorescent images were subjected to a rolling-ball background correction and the previously obtained particle positions were used to extract the median bead pixel intensity for a given concentration using image processing algorithms.

Devices with a minimum channel height of 2.2 µm and containing pre-trapped 2.8 µm BSA-functionalized beads were used to perform a proof-of-principle immunoassay in diluted whole blood (1:40). More specifically, the diluted, EDTA-treated rabbit whole blood (Envion) was spiked with varying concentrations of rabbit anti-BSA antibodies (Bethyl Laboratories; Bovine Albumin Polyclonal Antibody, A10-127A).

As secondary detection antibodies, Cy5-conjugated donkey anti-rabbit antibodies (JacksonImmuno, AB_2340607) were added to the spiked samples at 50 nM immediately before filling the capillary devices. The experiment was repeated three times for each anti-BSA antibody concentration. Six minutes after device filling, a fluorescent and a bright-field image of the trapping region was taken. The latter enabled easy identification of the beads via the same Python script described in the previous section. As a comparison, the same experiment was performed in PBS, devoid of any whole blood. The median fluorescence per bead was fit with a sigmoidal function, which was used to calculate the limit-of-detection (LOD) as the median signal of the control sample that is devoid of anti-BSA antibodies (blank) plus three times its standard deviation.

The 3D profile of the device was slightly adjusted to a minimum channel height of 1.9 µm to enable multiplexed detection of two different antigens in a proof-of-principle setting. More specifically, in addition to the 2.8 µm BSA-functionalized beads, 2 µm functionalized beads (Creative Diagnostics, WHM-G187) were similarly functionalized by incubating them in a PBS solution containing 25 pmol of biotinylated HRP (Thermo Fischer Scientific, 29139) per µg beads. After functionalization, the particles were incubated with a 0.9 mg/mL free biotin (Thermo Fischer Scientific, 29129) at room temperature for 30 min at 600 rpm to reduce cross-particle aggregation. Hereafter, both bead suspensions were added together and pre-trapped in the device as previously described. Subsequently, 50 nM Cy5 donkey anti-rabbit antibody was added to EDTA-treated rabbit whole blood (1:40 in PBS). The latter was spiked with rabbit anti-BSA and/or rabbit anti-HRP at 130 nM to showcase different immunological outcomes. Immediately afterwards, a 2 µl droplet of the solution was applied to the inlet of the capillary microfluidic device and left to flow for 6 min. The bead fluorescence at the relevant trapping lines was imaged using a Leica DMi8 equipped with a 40x objective (NA: 0.95) and numerically analyzed with a custom Python script.

By varying the cross-sectional area of the channels, such as the channel height, inside a microfluidic device, many new functionalities can be obtained, such as advanced flow focusing and on-chip particle size determination. One has previously exploited the effect of topographical variations inside fluidic channels to develop a capillary-driven device that can geometrically trap particles in the submicron regime. This effect was exploited to enable multiplexed antibody detection in patient serum. Nevertheless, the previously reported process required samples to undergo various processing steps outside the device, including serum incubation, secondary antibody labeling, washing. Moreover, due to the submicron channel height, the device was incompatible with whole blood samples.

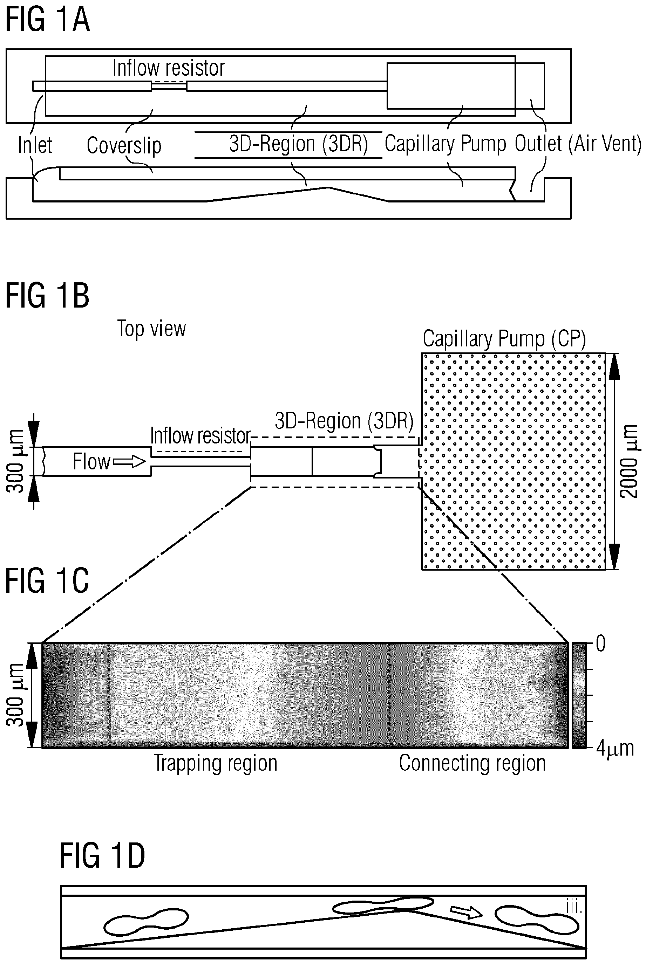

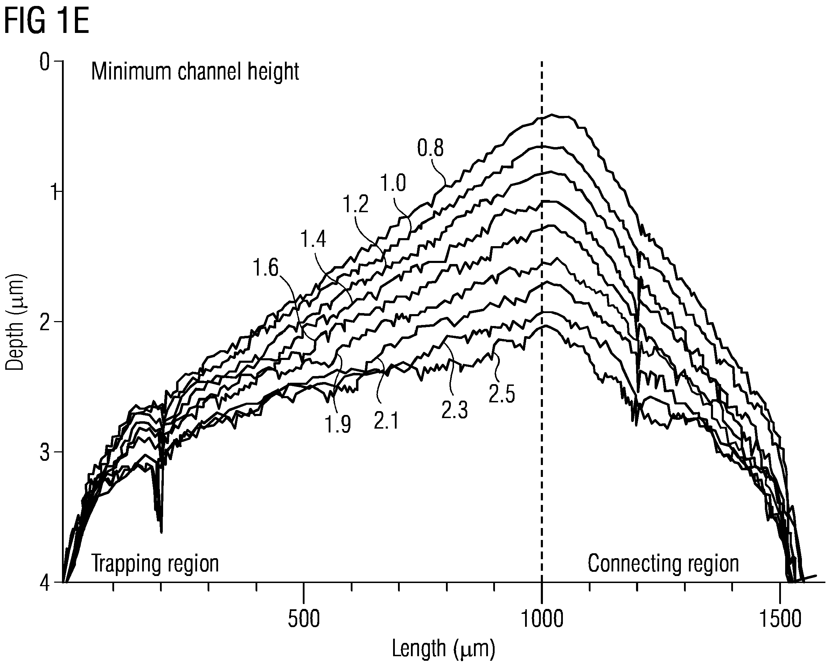

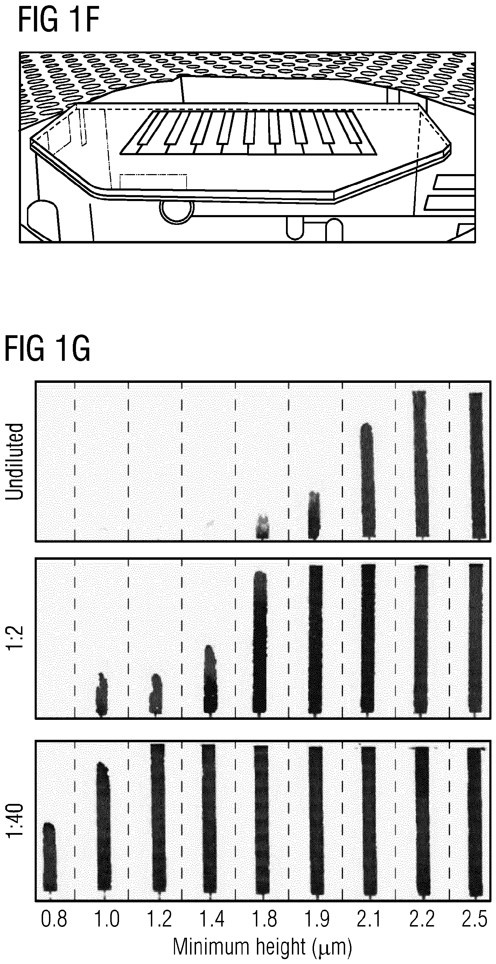

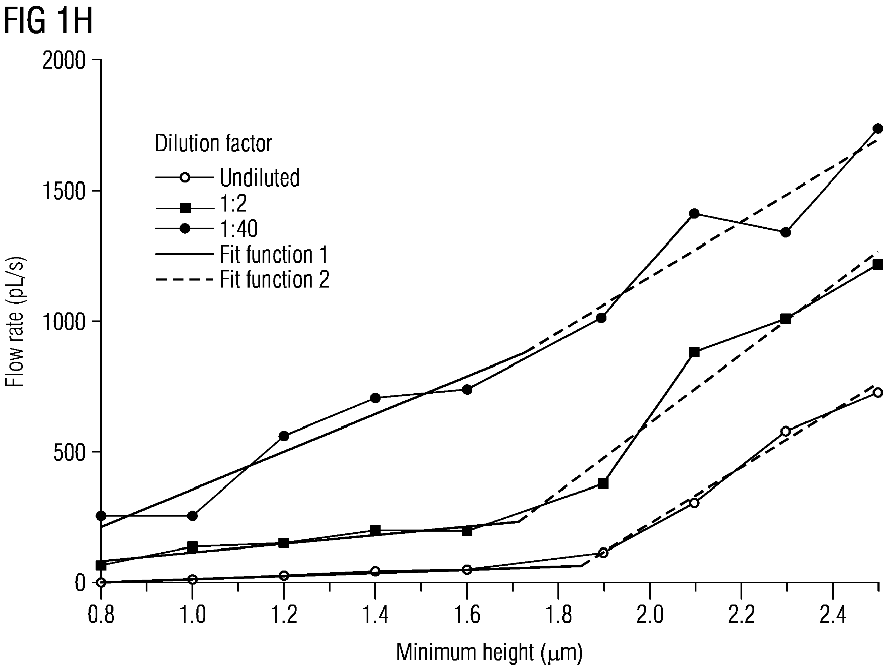

Figure 1 now shows in detail the influence of the channel topography in the capillary device on whole blood capillary flow:
Figure 1(a) schematically shows the different microfluidic components of the capillary device. The top section shows a top view and the bottom section a cross-section to highlight the change in channel topography, particularly in the diminishing cross-sectional area of the flow paths in the device as seen in the flow direction of the fluid.
Figure 1(b) represents a bright-field image of a bonded microfluidic device, showing the flow direction as well as the 3-dimensional region (3DR).
Figure 1(c) shows a confocal micrograph of the three-dimensional topography in the 3DR of the device with an outflow height of 800 nm.
Figure 1(d) depicts a graphical illustration of the three phases a red blood cell undergoes in the channel: i. normal flow ii. compression in shallow region iii. decompression in the capillary pump region.
Figure 1(e) shows the profiles of the 9 different wedge topographies which were used to find the ideal outflow height. The trapping region indicates where the particle immobilization takes place. The connecting region is representative of the tapering that connects the trapping region with the capillary pump.
Figure 1(f) is a photograph showing a PMMA-based chip containing 9 different devices with different channel topography on top of a 4" Si-wafer as a reference
Figure 1(g) shows a fluorescence image of whole blood (undiluted, 1:2, 1:40 in PBS) flowing through devices with different outflow heights. The shown channels have a width of 2000 µm.
Figure 1(h) represents the filling velocity of the capillary pump region at different concentrations of whole blood and for various outflow heights. The data was fit with a segmental linear regression fit. The two fit functions are given by the dotted and non-dotted black lines.

Thus, the device geometry has been adapted, resulting in a self-powered 3D microfluidic device comprising an inlet, inflow resistor (I.R.), an adapted 3D-region (3DR) and a capillary pump (Fig. 1a). During the execution of the method according to the present invention, in the capillary device, biofunctionalized particles are, first, size-dependently immobilized and then the device is vacuum dried. This enables on-chip immunoassays without any incubation steps outside of the device. Once dried, a 2 µl sample droplet can be applied at a 300 µm inflow channel at a depth of 4 µm (Fig. 1b-left). Here, due to surface tension, the sample will be aspirated into the channel. Subsequently, the sample will flow through an I.R., which is 100 µm wide and 500 µm long. These dimensions increase the fluidic resistance and ensure a more homogeneous filling front. The I.R. is succeeded by the 3DR, which comprises two topographically distinct sections.

Firstly, the channel height decreases over 1 mm to ensure size-dependent immobilization of the bio-functionalized particles. The second section of the 3DR brings the channel height back to 4 µm over a distance of 0.5 mm (Fig. 1c) and couples the active region of the device to a capillary pump (CP) (Fig. 1b - right). The CP is 4 µm deep, 2 mm wide and 22.5 mm long, resulting in a total filling capacity of ~150 nL. It contains pillars with a diameter of 30 µm and a pitch of 75 µm that contribute to flow control during the on-chip immunoassay.

Adapting the 3D channel profile for uninterrupted flow of whole blood

In order to enable whole blood assays to be easily performed on chip, it is of paramount importance that RBCs do not get halted in the 3DR. When they flow through the aforementioned section, they undergo three phases. Firstly, they flow freely (Fig. 1d (i)), after which they gradually compress and expand laterally until the shallowest point of the 3DR is reached (Fig. 1d (ii)). Subsequently, if they do not get stuck at this position, they will continue to flow to the capillary pump and decompress to their normal shape (Fig. 1d (iii)). The region where cellular compression takes place is defined as the trapping region (T.R.) and where decompression occurs is termed the connecting region (C.R). To identify the appropriate height profile of the device, which would prevent RBC blockage, nine devices were patterned with changing 3DRs. More specifically, the shallowest point was systematically decreased in ≈ 0.2 µm increments, while simultaneously retaining a maximum depth of 4 µm in the inflow section (Fig. 1e).

These nine devices were patterned on the same chip by grayscale e-beam lithography followed by nanoimprint lithography into a PMMA sheet (Fig. 1f). To enable pre-loading of bio-functionalized beads and to increase the device's hydrophilicity, the surfaces of both a patterned and unpatterned PMMA sheet were blocked with poly(vinyl alcohol) before sealing the device through UV/O-assisted thermal bonding . Afterwards, the devices were loaded with whole blood at different concentrations and the filling fraction in the capillary pump region was monitored after 5 min (Fig. 1g).

Here, it becomes noticeable that undiluted blood does not reach the capillary pump region in the devices with a shallower 3DR due to clogging effects. A qualitative change in the filling fraction of undiluted whole blood can be seen between channels with a minimum depth of 1.6 and 1.9 µm. When an increased dilution is used, this transition in filling fraction is shifted towards smaller minimum channel heights. To monitor this effect more quantitatively, a time lapse series was acquired at the beginning of the capillary pump region to monitor the initial filling behavior of the devices (Fig. 1h).

The obtained data were fit with a segmental linear regression to obtain an estimation of the transition height between flow regimes. For more concentrated blood samples, the major transition in filling rate occurs between 1.6 and 1.9 um. For a more dilute sample, the flow rate seems to decrease linearly dependent on the minimum channel height. This is highly likely due to an increase in the fluidic resistance caused by narrower channels. It is interesting to note that the aforementioned transition point is around the minimal reported red blood cell thickness of 1.7 µm. The described results highlight the importance of the right channel geometry in conjunction with the appropriate whole blood dilution to ensure unhindered loading of the 3D microfluidic device. To this extent, a new chip containing 9 devices of an equal minimal outflow height of 2.2 µm was fabricated.

### Preparation of 3D microfluidic devices to enable on-chip immunoassays

As previously mentioned, LFAs are attractive POC tools because of their rapid readout and the small, required sample volume. Yet, they only provide a binary answer and are characterized by limited sensitivity. Alternatively, laboratory-based enzyme-linked immunosorbent (ELISA) assays allow for a high degree of sensitivity and biomarker quantification. Nevertheless, a major shortcoming is their labor-intensive and time-consuming protocol. For example, a classical sandwich ELISA requires overnight plate functionalization and numerous consecutive sample loading steps with extensive intermediate washing. It is estimated that such an assay takes 5-7 hours for an experienced operator to perform. Moreover, ELISAs require a minimum sample volume of 100 µL per well or condition when using conventional 96 well plates, excluding sample volume required for possible duplicate or triplicate testing. This is not optimal, as in many life-science research fields, such as structural biology or high-throughput drug discovery, sample volumes are a limiting factor.

To address the above-mentioned shortcomings of both POCs and ELISAs, here the optimized 3D microfluidic device is used to perform on-chip immunoassays directly in whole blood. This device allows for rapid readout and analysis of small sample volumes, while at the same time it avoids numerous samplehanding steps. The immunoassay was structured similarly to an indirect ELISA. In these assays the antigen is prefunctionalized on the plate's surface, and the concentration of the antigen-specific primary antibody in the sample is determined using a secondary antibody conjugated with an enzyme.

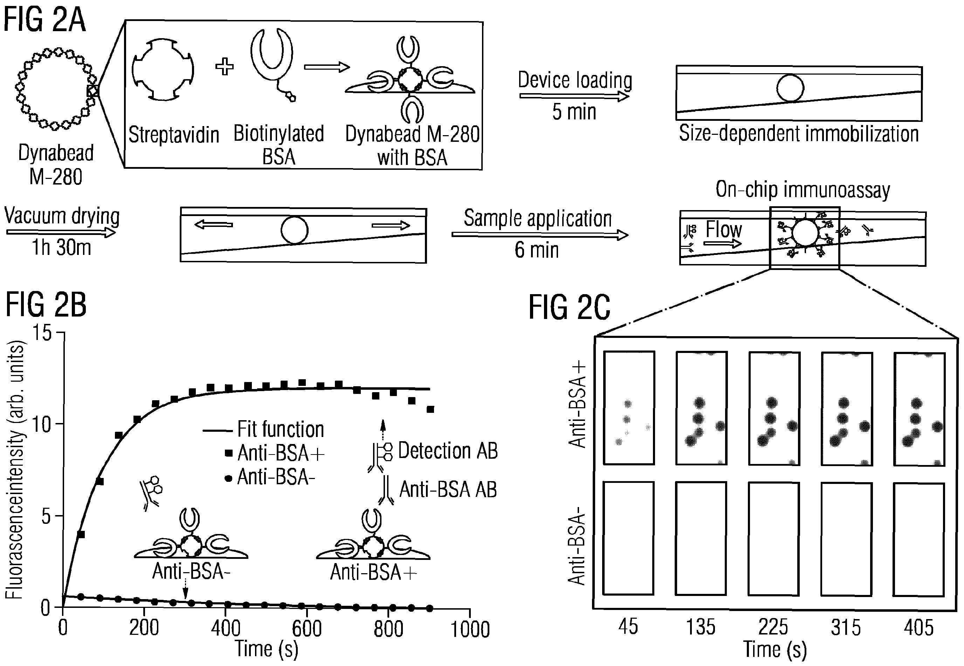

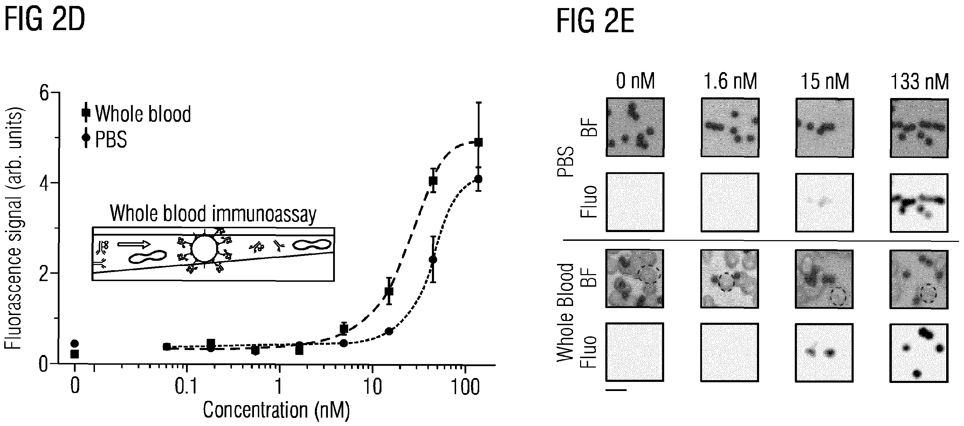

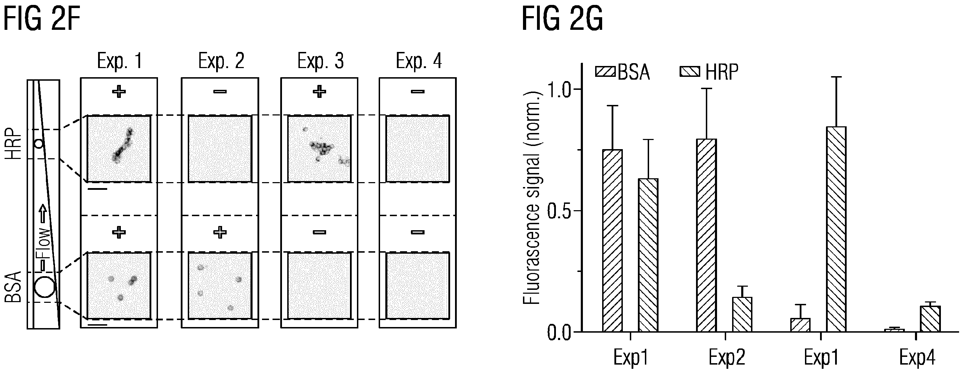

Figure 2 now schematically shows the principles of whole blood rapid and multiplexed immunoassays:
Figure 2(a) is a schematic illustration of the bead biofunctionalization as well as the pre-loading procedure of the particles inside the device.
Figure 2(b) gives the time lapse showing change in fluorescent signal of the trapped particles over time, both for BSA+ and BSA- samples.
Figure 2(c) shows fluorescent micrographs of BSA+ and BSAsamples at different times after device loading.
Figure 2(d) depicts a limit-of-detection curve showing the fluorescent intensity for different concentrations of anti-BSA antibody in whole blood (red dotted line) and PBS (orange dotted line).
Figure 2(e) shows bright-field and fluorescent micrographs of biofunctionalized particles in the TR when loaded with different concentrations of anti-BSA antibody in PBS (top) or whole blood (bottom). The red blood cells are outlined by a dashed line.
Figure 2(f) represents fluorescent images of 2 µm HRP and 2.8 µm biofunctionalized particles in different experimental conditions to show the multiplexing capabilities of the 3D microfluidic device.
Figure 2(g) provides the quantified fluorescent signal from particles shown in (f). The error bars represent the standard error of the mean (N = 3). All the scale bars represent 10 µm.

In the present case, 2.8 µm streptavidin-coated particles are functionalized with biotinylated bovine serum albumin, acting as an antigen (BSA; Fig. 2a). This was done in phosphate buffered saline (PBS) at a pH of 7.4. Afterwards, the functionalized beads were diluted in distilled water and loaded into the device. Once the capillary pump region was filled, the loaded devices were left to dry in vacuum for 1.5 h. Distilled water rather than PBS was used for the final particle dilution to prevent the formation of salt crystals and ensure proper and homogenous drying of the device. The pre-loading of the functionalized beads is similar to the overnight plate functionalization in indirect ELISAs but requires almost 23 hours less time, while retaining a high degree of antibody stability.

### Rapid one-drop detection of anti-BSA antibodies directly in whole blood

These devices with preloaded particles allows for rapid on-chip immunoassays. As a proof-of-principle experiment, a 2 µl PBS droplet containing diluted rabbit anti-BSA antibody as well as Cy5-conjugated donkey anti-rabbit antibody was applied onto the inflow region of the device. The former acts as a primary antibody against the antigen target, whereas the latter functions as a secondary fluorescent detection antibody. Hereafter, the liquid was passively aspirated into the fluidic channel and flowed over the pre-immobilized BSA functionalized particles. Due to the large surface-to-volume ratio, attributable to the shallow device dimensions as well as the spherical bead geometry, mass transport is increased and diffusion times are considerably reduced in comparison to ELISA. This enables rapid binding of the primary anti-BSA antibodies to its antigen.

Simultaneously, the secondary anti-rabbit antibodies conjugated with a fluorescent dye will bind to the primary antibodies on top of the beads. This enables indirect fluorescent detection of the binding event between the primary antibody and its antigen. The accumulation of the fluorescent signal on the beads was monitored by means of a time-lapse series (Fig. 2b). The signal steadily increased over time, reaching a maximum of around 360 s.

When visually comparing the different fluorescent images (Fig. 2c), the intensity difference between positive and negative control samples could already be seen after 45 seconds. Additionally, in the negative control experiment, the fluorescent signal on the particles did not increase over time, evidencing the absence of non-specific binding of the secondary antibody to the bead surface. On the contrary, a slight decrease over time can be seen. This can be explained by partial bleaching of the autofluorescence of the bead, some minor defocusing during time-lapse acquisition as well as bleaching of the unbound secondary antibodies inside the fluidic channel. The bleaching effect also becomes apparent after around 10 min in the case of the BSA-positive control sample. However, it should be mentioned that based on the previously acquired flow rates, the capillary pump is estimated to be filled after around 100 s, whereas the maximum signal was seen after 360 s. This could imply that not all the available binding sites on the bio-functionalized particles are saturated. For future considerations, it is worth adapting the capillary-pump region to increase the volume of analyte that is flushed over the beads, and therefore the number of primary antibodies on top of the beads' surface.

Also, it is important to note that because of device operation, no washing steps were performed, and unbound fluorescent detection antibodies remained in the channel. Consequently, the background signal increases, and could potentially mask the fluorescent signal on top of the bead's surface. Other microfluidic assays have addressed this issue by additional washing steps or complex pre-deposition antibodies to decrease the background signal over time and enable signal readout. However, this can be cumbersome and time-consuming.

The presented device inherently mitigates this issue because of its shallow dimensions and small detection volume. This ensures that the fluorescent signal in a given region of interest is primarily dominated by the upconcentrated fluorescent detection antibodies on the biofunctionalized particles, rather than the background.

### On-chip immunoassays reveal a 2 nM limit-of-detection

In the previous subsection, it was shown that it is possible to rapidly detect the presence of BSA specific antibodies in a physiological buffer solution. As a next step, the whole blood assay was benchmarked against its limit-of-detection in PBS. To do so, the lowest detectable concentration of rabbit anti-BSA antibody was determined, while keeping the concentration of secondary detection antibodies constant (Fig. 2d). Each sample was left to flow over the pre-immobilized beads for 6 min to ensure maximal binding of all the assay constituents. Afterwards, bright-field and fluorescent micrographs of the beads in the trapping region were acquired and the corresponding fluorescence intensity was quantified (Fig. 2e-top) .

The quantified data revealed an LOD of 11 nM in PBS. The same experiment was repeated for diluted whole blood (1:40) and revealed an ameliorated LOD at 2 nM. This discrepancy can be possibly attributed to the blocking effect of serum constituents, which reduces non-specific binding interactions. This is evidenced by the higher signal of the PBS control in comparison to that in whole blood. Nevertheless, the achieved LODs, both for PBS and whole blood, are in the relevant range for a variety of analytes and disease-specific antibodies.

The bright-field micrographs of the whole blood samples (Fig. 2e - bottom) show that even though the hematocrit is drastically reduced by the increased dilution, RBCs are still present in the microfluidic channels. At this point, it is worth emphasizing that due to geometrical size constraints, the RBCs can't cover the pre-immobilized particles on their top and bottom surfaces. This means that from a top view, the RBCs cannot conceal the beads, which ensures that the fluorescent signal originating from the surface of the beads is not diminished by possible scattering and absorption effects. Additionally, it should be mentioned that due to the small height in the trapping region, it is not possible for multiple RBCs to stack on top of each other, further reducing the influence of signal diminishing effects. These experiments highlight that the LOD of BSA-specific antibodies is not negatively impacted by the presence of diluted whole blood.

### Passive particle size-separation enabling facile two-fold multiplexed antibody detection

The passive size-dependent particle immobilization of the device has previously been shown to enable on-chip multiplexed detection of antibodies with distinct antigen targets. Here, It is shown that similar assays are possible using whole blood assays. To do so, the shallowest point of the device was slightly reduced from 2.2 µm to 1.9 µm. This decrease in minimum channel outflow height enabled the trapping of smaller, 2 µm particles. The latter were functionalized with biotinylated HRP added to a suspension consisting of 2.8 µm BSA functionalized beads. Subsequently, both particles were pre-trapped in the trapping section of the 3DR, as explained previously.

As a proof-of-principle experiment, diluted whole blood samples (1:40) containing controlled combinations of rabbit anti-HRP and rabbit anti-BSA antibodies were loaded into the device. The evaluation of the binding event of the primary antibodies with their respective antigen targets on the different bead sizes was enabled by the addition of a Cy5-conjugated fluorescent anti-rabbit detection antibody. After an incubation time of 6 min, high magnification fluorescent micrographs were taken at the expected positions inside the trapping section (Fig. 2f). From these images, it becomes clear that the fluorescence intensity at the edges of the biofunctionalized particles is greater than the intensity in the middle. This is to be expected, as the particles are geometrically immobilized by the tapered wedge profile inside the fluidic channel on their top and bottom surfaces.

Consequently, the largest interaction between the primary antibodies and their antigen targets occurs on the bead's surface facing the volume of the channel.

The use of biofunctionalized particles increases the surface-to-volume ratio and the associated reaction field. Consequently, the diffusion distance is even further reduced, possibly increasing reaction rates. The quantification of the bead's fluorescent signal is in agreement with the presence or absence of the respective primary antibodies (Fig. 2g). More specifically, in exp. 1 (BSA+/HRP+), a large fluorescent signal can be seen at both pre-trapped biofunctionalized particles. However, when either the primary HRP (exp. 2) or BSA (exp. 3) antibody is removed, the obtained fluorescent signal at the relative trapping position drops sharply. If both primary antibodies are removed and only whole blood spiked with fluorescent detection antibodies is loaded into the device (exp. 4), the signal at the trapping positions drops even further.

This proof-of-principle experiment highlights that by using distinct biofunctionalized particles of different sizes, it is possible to perform on-chip multiplexed antibody detection against different antigen targets or diseases in whole blood samples. This while retaining a low sample volume and rapid readout. The reported method can deliver the results on a similar timescale as LFAs, but provide the possibility for semi-quantitative signal analysis, which is in sharp contrast to the binary result which is obtained by the aforementioned POC devices.

The results presented in this application show that it is possible to perform methods for immunoassays directly in whole blood by using capillary-driven microfluidic devices with a changing channel topography. The 3D profile inside the microfluidic channel proved to have a critical impact on the filling behavior of both undiluted and diluted whole blood samples. More specifically, it was found that a minimum channel height of 1.4 µm is required to enable facile passage of the RBCs through the trapping region and to reach the capillary pump. It was shown that an optimized 3DR enables efficient pre-trapping of biofunctionalized particles and due to the high surface-to-volume ratio, sub-10 min sample readout can be achieved. Moreover, it is highlighted that the presence of whole blood does not negatively affect the limit-of-detection when compared to standard PBS. An adapted version of the 3DR allowed for concurrent pre-trapping of two different particle sizes, each functionalized with a different antigen. This permitted twofold multiplexing in a series of proof-of-principle experiments, while retaining very low volume requirements, rapid sample readout and quantitative analysis. Of course, even higher multiplexing (than twofold) can be possible by adjusting the particles size and the device's channel widths accordingly.

The present invention further reduces the limit-of-detection. This can be done in a variety of ways. One could increase the total volume of the capillary pump in an effort to increase the number of bound antibodies on the biofunctionalized particles. Also, the use of brighter and more photostable fluorophores, such as quantum dots or the addition of antifading chemicals could potentially lead to a better signal-tonoise ratio.

Furthermore, even though in the current paper only twofold multiplexing is presented, it should be emphasized that this can easily be extended by several strategies. More specifically, the use of color-multiplexing has been previously reported for the simultaneous detection of various antibody types on one single biofunctionalized particle. In addition, it would be possible to use multiple channels within a given microscopic field-of-view. This form of parallel multiplexing can be complemented by increasing the number of particle sizes pre-trapped in the microfluidic channels. Thus, many more analytes could be concurrently analyzed with one single fluorescent micrograph.

On the device fabrication side, alternate combinations of nanofabrication methods are possible to render the entire process even more cost-effective and upscalable. For instance, one could go from a 4-inch process to an 8-inch one and replace hot-embossing with other high-throughput methods, such as roll-to-roll embossing or injection molding.

The present method using the 3D microfluidic device further extends the use of the previously developed 3D nanofluidic device towards whole blood assays. For other research endeavors, it is easily possible to adapt the fluidic trapping section to various bio-fluids, such as saliva or urine, to increase the method and the device's versatility. Subsequently, by combining this with smartphone fluorescent microscopy, the technology makes a meaningful impact on the biomarker detection landscape.

## Claims

1. A method for rapid immunoassays using a three-dimensional microfluidic capillary system, said method comprising the steps of:
a) providing the 3D microfluidic capillary system having a 3D fluidic trapping section having its cross-section area diminishing in the flow direction of the fluid through the system,
b) providing an ensemble of support beads being covered with a functionalized layer or providing biofunctionalized particles, wherein the support beads or the biofunctionalized particles having a dimension to enable them to be size-dependently immobilized at a predefined position in the fluidic trapping section and wherein the functionalized layer or the biofunctionalized particles are chosen to bind one or more specimens of biochemical compounds;
c) conveying the support beads or the biofunctionalized particles into the fluidic trapping section thereby immobilizing the support beads or the biofunctionalized particles at the predefined position;
d) conveying subsequently a probe fluid comprising various specimens of biochemical compounds through the fluidic trapping section, wherein one or more of the various specimens bind to the functionalized layer or to the biofunctionalized particles;
e) conveying optionally a buffer fluid to flush the channels to remove the excessive unbound molecules
and
f) detecting qualitatively or quantitatively the amount of the one or more specimens that are bound to the functionalized layer being immobilized with the support beads bearing said functionalized layer or to the immobilized biofunctionalized particles.

2. The method according to claim 1, wherein the fluidic trapping section is dried after the trapping of the support beads or of the biofunctionalized particles.

3. The method according to claim 1 or 2, wherein the specimen of biochemical compound is a peptide, lipide and/or a protein and/or an antibody.

4. The method according to any of the preceding claims, wherein the probe fluid is whole blood.

5. The method according to any of the preceding claims wherein distinct biofunctionalized particles or layered support beads of different sizes, thus enabling an on-chip multiplexed antibody detection against different antigen targets or diseases in the sample fluid, preferably in whole blood samples.
